# EUROPEAN PATENT APPLICATION

(11) **EP 3 183 965 A1**
(43) Date of publication of application: **28.06.2017**
(21) Application number: 15202519.3
(22) Date of filing: 23.12.2015
(51) Int. Cl.: A01N 25/04, A01N 43/90, A01N 59/06, A01N 65/20

(54) **COMPOSITION FOR AQUATIC PEST CONTROL**

(71) Applicant: Omya International AG, 4665 Oftringen (CH)
(72) Inventor: GANTENBEIN, Daniel, 4056 Basel (CH); DIAZ QUIJANO, Carolina, 4665 Oftringen (CH); SCHOELKOPF, Joachim, 5727 Oberkulm (CH); GANE, Patrick A. C., 4852 Rothrist (CH)
(74) Representative: Müller-Dyck, Martina

(57) **Abstract**

The present invention relates to a composition comprising an aquatic pesticide and a surface-reacted calcium carbonate, wherein the aquatic pesticide comprises rotenone, and the surface-reacted calcium carbonate is a reaction product of natural ground calcium carbonate or precipitated calcium carbonate with carbon dioxide and one or more H₃0⁺ ion donors, wherein the carbon dioxide is formed in situ by the H₃O⁺ ion donor treatment and/or is supplied from an external source.

## Description

The present invention relates to the field of pest control products, and especially to a composition for controlling an aquatic pest, its use and a method of producing the same as well as a method of controlling a pest.

Rotenone is a natural toxin produced by several tropical plants and has been used for centuries as a selective fish poison. It is highly toxic to fish and other aquatic life, but has low toxicity to birds and mammals. Rotenone is non-persistent in the environment, being quickly broken down by light and heat. It does not accumulate in animals and is readily metabolised and excreted. Rotenone is now recognised as the most environmentally benign of the commonly used fish poisons (piscicides or ichthyocides) and remains extremely useful for the chemical rehabilitation of fish habitats to remove noxious species and for research sampling (cf. Nicholas Ling, "Rotenone - a review of its toxicity and use for fisheries management", Science for Conservation, 2002, 211, page 6).

Furthermore, rotenone was used to treat fish parasites in rivers and aquacultures, for example, the invasive ectoparasite *Gyrodactylus salaries.* Said ectoparasite appeared in Norway in 1975 and is considered one of the worst threats to native Atlantic Salmon *Salmo salar,* causing epidemics that have reduced parr density and returning adults by 86-87% in 48 Norwegian rivers. It was tried to eradicate the parasites through host removal wherever possible and reduce their numbers where eradication is not possible. The eradication efforts in the last 30 years have led to a wide range of improved methodologies being developed, such as better strategies based on continuous knowhow generation, better equipment, or improvement of the chemical formulation which contains the fish toxicant rotenone.

However, there is an important challenge connected to ground water in rivers. Small juvenile salmon often stay along the river beds and among gravel in small brooks and seeps, where significant outlets of ground water are often found. Groundwater contains no rotenone and, therefore, upwelling of groundwater between stones and gravel can allow these small salmon to escape the treatment and may later lead to a re-infection of the treated river.

A further worldwide problem in aquatic ecosystems is the intentional or authorized as well as unauthorized introduction of invasive alien species. Many of these species are causing economic and environmental problems, such as reduced biodiversity. Globally, invasive alien species have been recognised by the International Union for Conservation of Nature (IUCN) as the second greatest threat to biodiversity after habitat loss. The EU regulation 1143/2014 recommends rapid actions such as eradication of invasive alien species.

US 5,674, 518 A describes a piscicide formulation, which is combined with fish feed and a target species attractant to form poison-doped oral bait pellets.

US 3,761,238 A relates to a particulate composition for application in water environment comprising an inert particulate carrier, the surface of the particles having inherent or extrinsic ion-exchange characteristics, and a normally water-soluble toxicant reversibly absorbed in ionic form upon said surfaces.

US 2012/0295790 A1 relates to a pesticidal composition comprising microcapsules which contain a pesticidal active ingredient and a suitable carrier and to a method of controlling pests comprising the application of an effective amount of such a pesticidal composition within a locus where pests are or are expected to be present. Said microcapsules exhibit sustained-release properties. Likewise,
WO 2010/037753 A1 discloses a controlled release active agent carrier. Said carrier comprises a surface-reacted natural or synthetic calcium carbonate and one or more active agents.

However, there remains a need in the art for formulations for controlling aquatic pests.
Accordingly, it is an object of the present invention to provide a composition which can control an aquatic pest, and especially a pest fish or a fish parasite. It would be desirable to provide a composition with improved handling properties and efficiency. It would also be desirable that said composition is not persistent in the environment. Furthermore, it would be desirable that the composition also allows the use of hydrophobic or water-insoluble active agents or pesticides.

A further object of the present invention is the provision of a composition which can be applied in a specifically targeted way, remains at the application area, and allows better control of the action site. It would also be desirable if said composition could be applied less frequently and/or reduces the workload during application. It would also be desirable that it is easy to apply and provides a good or improved efficiency compared to compositions of the prior art.

The foregoing and other objects are solved by the subject-matter as defined herein in the independent claims.

According to one aspect of the present invention, a composition comprising an aquatic pesticide and a surface-reacted calcium carbonate is provided, wherein
the aquatic pesticide comprises rotenone, and
the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source.

According to another aspect of the present invention, a method for preparing a composition according to the present invention is provided, comprising the following steps:
(I) providing a solution of an aquatic pesticide, wherein the aquatic pesticide comprises rotenone,
(II) providing a surface-reacted calcium carbonate, wherein the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source, and
(III) mixing the solution of step (I) and the particulate material of step (II).

According to still another aspect of the present invention, use of a composition for controlling an aquatic pest is provided,
wherein the composition comprises an aquatic pesticide and a surface-reacted calcium carbonate, and
wherein the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source.

According to still another aspect of the present invention, a method of controlling an aquatic pest is provided,
wherein the method comprises the step of applying a composition comprising an aquatic pesticide and a surface-reacted calcium carbonate onto at least a part of an aquatic system, and
wherein the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source.

Advantageous embodiments of the present invention are defined in the corresponding sub-claims.

According to one embodiment the aquatic pesticide has an absolute water solubility at 20°C of less than 10 g/l, preferably less than 1 g/l, more preferably less than 0.1 g/l, and most preferably less than 0.01 g/l. According to another embodiment the aquatic pesticide and the surface-reacted calcium carbonate are present in a weight ratio from 0.1:1 to 20:1, preferably from 1:1 to 15:1, more preferably from 1.5:1 to 10:1, and most preferably from 2:1 to 5:1. According to still another embodiment the aquatic pesticide is selected from the group consisting of rotenone, a rotenone-containing plant extract, a formulation comprising rotenone, and mixtures thereof, preferably the aquatic pesticide is selected from the group consisting of rotenone, cube resin, derris resin, CFT Legumine, TIFA Chem Fish, Prentox Prenfish, Cuberol, Fish Tox, Noxfire, Rotacide, Sinid, Tox-R, Curex Flea Duster, Derrin, Cenol Garden Dust, Chem-Mite, Cibe Extract, Green Cross Warble Powder, and mixtures thereof.

According to one embodiment the surface-reacted calcium carbonate has a volume median particle size *d*₅₀ from 1 to 75 µm, preferably from 2 to 50 µm, more preferably from 3 to 40 µm, even more preferably from 4 to 30 µm, and most preferably from 4 to 15 µm. According to another embodiment the surface-reacted calcium carbonate is obtained by a process comprising the steps of: (a) providing a suspension of natural ground calcium carbonate or precipitated calcium carbonate, (b) adding at least one acid having a pKₐ value of 0 or less at 20°C or having a pKₐ value from 0 to 2.5 at 20°C to the suspension of step a), and (c) treating the suspension of step (a) with carbon dioxide before, during or after step (b). According to still another embodiment the surface-reacted calcium carbonate is obtained by a process comprising the steps of: (A) providing a natural ground calcium carbonate or precipitated calcium carbonate, (B) providing at least one water-soluble acid, (C) providing gaseous carbon dioxide, (D) contacting said natural ground calcium carbonate or precipitated calcium carbonate of step (A) with the at least one acid of step (B) and with the carbon dioxide of step (C), characterised in that: (i) the at least one acid of step (B) has a pKₐ greater than 2.5 and less than or equal to 7 at 20°C, associated with the ionisation of its first available hydrogen, and a corresponding anion is formed on loss of this first available hydrogen capable of forming a water-soluble calcium salt, and (ii) following contacting the at least one acid with natural ground calcium carbonate or precipitated calcium carbonate, at least one water-soluble salt, which in the case of a hydrogen-containing salt has a pKₐ greater than 7 at 20°C, associated with the ionisation of the first available hydrogen, and the salt anion of which is capable of forming water-insoluble calcium salts, is additionally provided.

According to one embodiment the natural ground calcium carbonate is selected from the group consisting of marble, chalk, limestone, and mixtures thereof, or the precipitated calcium carbonate is selected from the group consisting of precipitated calcium carbonates having an aragonitic, vateritic or calcitic crystal form, and mixtures thereof. According to another embodiment the composition is in form of an aqueous suspension, preferably having a solid content from 1 to 85 wt.-%, more preferably from 5 to 50 wt.-%, and most preferably from 10 to 25 wt.-%, based on the total weight of the aqueous suspension.

According to one embodiment the aquatic pest is a water plant, a crustacean, an invasive species, an insect, a nematode , a mollusc, a fish, a parasite and/or a pathogen, preferably a fish and/or parasite, more preferably a fish parasite, even more preferably a fish parasite selected from the group consisting of *Gyrodactylus salaris, Ichthyophthirius multifiliis*, cryptocaryon, velvet disease, *Brooklynella hostilis*, *Glugea*, *Ceratomyxa shasta*, *Kudoa thyrsites, Tetracapsuloides bryosalmonae*, *Cymothoa exigua*, flukes, carp lice, salmon lice, and mixtures thereof, and most preferably *Gyrodactylus salaries.* According to another embodiment the pest is controlled by controlling the host of the pest, wherein the host is preferably a fish. According to still another embodiment the aquatic system is a catch basin, a pond, a breeding tank, a lake, a bay, a wet land, a marsh, a swamp, a tidal basin, a lagoon, a storm water retention pond, a sound, a creek, a stream, a river, an ocean, a ditch, a swale, a sewage treatment system, a pothole, a tree hole, a rock hole, a water reservoir, a river, a watercourse, or a sewerage system.

It should be understood that for the purpose of the present invention, the following terms have the following meaning:
The expression "control(s) a pest" or "controlling a pest" as used herein comprises preventing, treating, reducing, eliminating, or eradicating a pest, inhibiting the rate or extend of a pest attack, or delaying the onset of a pest attack.

For the purpose of the present invention the term "aquatic pest" refers to any species, strain or biotype of plant, animal or pathogen injurious to animals and/or plants in an aquatic environment or aquatic ecosystem. Examples of aquatic pests are insects, nematodes, parasites, gastropods, fish, invasive species, water plants, crustaceans or pathogens such as fungi, viruses, or bacteria.

The term "particulate" in the meaning of the present application refers to materials composed of a plurality of particles. Said plurality of particles may be defined, for example, by its particle size distribution. The expression "particulate material" may comprise granules, powders, grains, tablets, or crumbles.

The term "solid" refers to a physical state of a material. Unless indicated otherwise, this physical state is to be observed at a temperature of 20°C.

For the purpose of the present application, "water-insoluble" materials are defined as those which, when mixed with 100 ml of deionised water and filtered at 20°C to recover the liquid filtrate, provide less than or equal to 0.1 g of recovered solid material following evaporation at 95 to 100°C of 100 g of said liquid filtrate. "Water-soluble" materials are defined as materials leading to the recovery of greater than 0.1 g of solid material following evaporation at 95 to 100°C of 100 g of said liquid filtrate. In order to assess whether a material is an insoluble or soluble material in the meaning of the present invention, the sample size is greater than 0.1 g, preferably 0.5 g or more.

"Natural ground calcium carbonate" (GCC) in the meaning of the present invention is a calcium carbonate obtained from natural sources, such as limestone, marble, or chalk, and processed through a wet and/or dry treatment such as grinding, screening and/or fractionating, for example, by a cyclone or classifier.

"Precipitated calcium carbonate" (PCC) in the meaning of the present invention is a synthesised material, obtained by precipitation following reaction of carbon dioxide and lime in an aqueous, semi-dry or humid environment or by precipitation of a calcium and carbonate ion source in water. PCC may be in the vateritic, calcitic or aragonitic crystal form. PCCs are described, for example, in EP 2 447 213 A1, EP 2 524 898 A1, EP 2 371 766 A1, EP 1 712 597 A1, EP 1 712 523 A1, or WO 2013/142473 A1.

The term "surface-reacted" in the meaning of the present application shall be used to indicate that a material has been subjected to a process comprising partial dissolution of said material upon acidic treatment (e.g., by use of water-soluble free acids and/or acidic salts) in aqueous environment followed by a crystallization process which may occur in the absence or presence of further crystallization additives. The term "acid" as used herein refers to an acid in the meaning of the definition by Brønsted and Lowry (e.g., H₂SO₄, HSO₄⁻), wherein the term "free acid" refers only to those acids being in the fully protonated form (e.g., H₂SO₄).

The "particle size" of particulate materials other than surface-reacted calcium carbonate herein is described by its distribution of particle sizes *dₓ*. Therein, the value *dₓ* represents the diameter relative to which x % by weight of the particles have diameters less than *dₓ*. This means that, for example, the *d*₂₀ value is the particle size at which 20 wt.-% of all particles are smaller than that particle size. The *d*₅₀ value is thus the weight median particle size, i.e. 50 wt.-% of all particles are smaller than this particle size. For the purpose of the present invention, the particle size is specified as weight median particle size *d*₅₀ unless indicated otherwise. Particle sizes were determined by using a Sedigraph^{™} 5100 instrument of Micromeritics Instrument Corporation. The method and the instrument are known to the skilled person and are commonly used to determine the particle size of fillers and pigments. The measurements were carried out in an aqueous solution of 0.1 wt.-% Na₄P₂O₇.

The "particle size" of surface-reacted calcium carbonate herein is described as volume-based particle size distribution. For determining the volume-based particle size distribution, e.g., the volume-based median particle size (*d*₅₀) or the volume-based top cut particle size (*d*₉₈) of surface-reacted calcium carbonate, a Malvern Mastersizer 2000 Laser Diffraction System with a defined refractive index (RI) of 1.57 and imaginary refractive index (iRI) of 0.005 and Malvern Application Software 5.60 was used. The measurement was performed with an aqueous dispersion. For this purpose, the samples were dispersed using a high-speed stirrer. The weight determined particle size distribution may correspond to the volume determined particle size if the density of all the particles is equal.

The "specific surface area" (expressed in m²/g) of a material as used throughout the present document can be determined by the Brunauer Emmett Teller (BET) method with nitrogen as adsorbing gas and by use of a Gemini V instrument from Micromeritics. The method is well known to the skilled person and defined in ISO 9277:1995. Samples are conditioned at 250°C for a period of 30 min prior to measurement. The total surface area (in m²) of said material can be obtained by multiplication of the specific surface area (in m²/g) and the mass (in g) of the material.

In the context of the present invention, the term "pore" is to be understood as describing the space that is found between and/or within particles, i.e. that is formed by the particles as they pack together under nearest neighbour contact (interparticle pores), such as in a powder or a compact and/or the void space within porous particles (intraparticle pores), and that allows the passage of liquids under pressure when saturated by the liquid and/or supports absorption of surface wetting liquids.

For the purpose of the present invention, the "solids content" of a liquid composition is a measure of the amount of material remaining after all the solvent or water has been evaporated. If necessary, the "solids content" of a suspension given in wt.-% in the meaning of the present invention can be determined using a Moisture Analyzer HR73 from Mettler-Toledo (T= 120°C, automatic switch off 3, standard drying) with a sample size of 5 to 20 g.

Unless specified otherwise, the term "drying" refers to a process according to which at least a portion of water is removed from a material to be dried such that a constant weight of the obtained "dried" material at 120°C is reached. Moreover, a "dried" or "dry" material may be defined by its total moisture content which, unless specified otherwise, is less than or equal to 1.0 wt.-%, preferably less than or equal to 0.5 wt.-%, more preferably less than or equal to 0.2 wt.-%, and most preferably between 0.03 and 0.07 wt.-%, based on the total weight of the dried material.

For the purpose of the present invention, the term "viscosity" or "Brookfield viscosity" refers to Brookfield viscosity. The Brookfield viscosity is for this purpose measured by a Brookfield DV-II+ Pro viscometer at 25°C ± 1°C at 100 rpm using an appropriate spindle of the Brookfield RV-spindle set and is specified in mPa·s. Based on his technical knowledge, the skilled person will select a spindle from the Brookfield RV-spindle set which is suitable for the viscosity range to be measured. For example, for a viscosity range between 200 and 800 mPa·s the spindle number 3 may be used, for a viscosity range between 400 and 1 600 mPa·s the spindle number 4 may be used, for a viscosity range between 800 and 3 200 mPa·s the spindle number 5 may be used, for a viscosity range between 1 000 and 2 000 000 mPa·s the spindle number 6 may be used, and for a viscosity range between 4 000 and 8 000 000 mPa·s the spindle number 7 may be used.

A "suspension" or "slurry" in the meaning of the present invention comprises undissolved solids and water, and optionally further additives, and usually contains large amounts of solids and, thus, is more viscous and can be of higher density than the liquid from which it is formed.

Where an indefinite or definite article is used when referring to a singular noun, e.g., "a", "an" or "the", this includes a plural of that noun unless anything else is specifically stated.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Terms like "obtainable" or "definable" and "obtained" or "defined" are used interchangeably. This, for example, means that, unless the context clearly dictates otherwise, the term "obtained" does not mean to indicate that, for example, an embodiment must be obtained by, for example, the sequence of steps following the term "obtained" though such a limited understanding is always included by the terms "obtained" or "defined" as a preferred embodiment.

Whenever the terms "including" or "having" are used, these terms are meant to be equivalent to "comprising" as defined hereinabove.

According to the present invention a composition comprising an aquatic pesticide and a surface-reacted calcium carbonate is provided. The aquatic pesticide comprises rotenone and the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source.

In the following preferred embodiments of the inventive composition will be set out in more detail. It is to be understood that these embodiments and details also apply to the inventive methods and uses.

### The surface-reacted calcium carbonate

The composition of the present invention comprises a surface-reacted calcium carbonate, wherein the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source.

An H₃O⁺ ion donor in the context of the present invention is a Brønsted acid and/or an acid salt, i.e. a salt containing an acidic hydrogen.

In a preferred embodiment of the invention the surface-reacted calcium carbonate is obtained by a process comprising the steps of: (a) providing a suspension of natural or precipitated calcium carbonate, (b) adding at least one acid having a pKₐ value of 0 or less at 20°C or having a pKₐ value from 0 to 2.5 at 20°C to the suspension of step a), and (c) treating the suspension of step (a) with carbon dioxide before, during or after step (b). According to another embodiment the surface-reacted calcium carbonate is obtained by a process comprising the steps of: (A) providing a natural or precipitated calcium carbonate, (B) providing at least one water-soluble acid, (C) providing gaseous CO₂, (D) contacting said natural or precipitated calcium carbonate of step (A) with the at least one acid of step (B) and with the CO₂ of step (C), characterised in that: (i) the at least one acid of step B) has a pKₐ of greater than 2.5 and less than or equal to 7 at 20°C, associated with the ionisation of its first available hydrogen, and a corresponding anion is formed on loss of this first available hydrogen capable of forming a water-soluble calcium salt, and (ii) following contacting the at least one acid with natural or precipitated calcium carbonate, at least one water-soluble salt, which in the case of a hydrogen-containing salt has a pKₐ of greater than 7 at 20°C, associated with the ionisation of the first available hydrogen, and the salt anion of which is capable of forming water-insoluble calcium salts, is additionally provided.

"Natural ground calcium carbonate" (GCC) preferably is selected from calcium carbonate containing minerals selected from the group comprising marble, chalk, limestone and mixtures thereof. Natural calcium carbonate may comprise further naturally occurring components such as magnesium carbonate, alumino silicate etc.

In general, the grinding of natural ground calcium carbonate may be a dry or wet grinding step and may be carried out with any conventional grinding device, for example, under conditions such that comminution predominantly results from impacts with a secondary body, i.e. in one or more of: a ball mill, a rod mill, a vibrating mill, a roll crusher, a centrifugal impact mill, a vertical bead mill, an attrition mill, a pin mill, a hammer mill, a pulveriser, a shredder, a de-clumper, a knife cutter, or other such equipment known to the skilled man. In case the calcium carbonate containing mineral material comprises a wet ground calcium carbonate containing mineral material, the grinding step may be performed under conditions such that autogenous grinding takes place and/or by horizontal ball milling, and/or other such processes known to the skilled man. The wet processed ground calcium carbonate containing mineral material thus obtained may be washed and dewatered by well-known processes, e.g. by flocculation, filtration or forced evaporation prior to drying. The subsequent step of drying (if necessary) may be carried out in a single step such as spray drying, or in at least two steps. It is also common that such a mineral material undergoes a beneficiation step (such as a flotation, bleaching or magnetic separation step) to remove impurities.

"Precipitated calcium carbonate" (PCC) in the meaning of the present invention is a synthesized material, generally obtained by precipitation following reaction of carbon dioxide and calcium hydroxide in an aqueous environment or by precipitation of calcium and carbonate ions, for example CaCl₂ and Na₂CO₃, out of solution. Further possible ways of producing PCC are the lime soda process, or the Solvay process in which PCC is a by-product of ammonia production. Precipitated calcium carbonate exists in three primary crystalline forms: calcite, aragonite and vaterite, and there are many different polymorphs (crystal habits) for each of these crystalline forms. Calcite has a trigonal structure with typical crystal habits such as scalenohedral (S-PCC), rhombohedral (R-PCC), hexagonal prismatic, pinacoidal, colloidal (C-PCC), cubic, and prismatic (P-PCC). Aragonite is an orthorhombic structure with typical crystal habits of twinned hexagonal prismatic crystals, as well as a diverse assortment of thin elongated prismatic, curved bladed, steep pyramidal, chisel shaped crystals, branching tree, and coral or worm-like form. Vaterite belongs to the hexagonal crystal system. The obtained PCC slurry can be mechanically dewatered and dried.

According to one embodiment of the present invention, the precipitated calcium carbonate is precipitated calcium carbonate, preferably comprising aragonitic, vateritic or calcitic mineralogical crystal forms or mixtures thereof.

Precipitated calcium carbonate may be ground prior to the treatment with carbon dioxide and at least one H₃O⁺ ion donor by the same means as used for grinding natural calcium carbonate as described above.

According to one embodiment of the present invention, the natural or precipitated calcium carbonate is in form of particles having a weight median particle size *d*₅₀ of 0.05 to 10.0 µm, preferably 0.2 to 5.0 µm, more preferably 0.4 to 3.0 µm, most preferably 0.6 to 1.2 µm, especially 0.7 µm. According to a further embodiment of the present invention, the natural or precipitated calcium carbonate is in form of particles having a top cut particle size *d*₉₈ of 0.15 to 55 µm, preferably 1 to 40 µm, more preferably 2 to 25 µm, most preferably 3 to 15 µm, especially 4 µm.

The natural and/or precipitated calcium carbonate may be used dry or suspended in water. Preferably, a corresponding slurry has a content of natural or precipitated calcium carbonate within the range of 1 wt.-% to 90 wt.-%, more preferably 3 wt.-% to 60 wt%, even more preferably 5 wt.-% to 40 wt.-%, and most preferably 10 wt.-% to 25 wt% based on the weight of the slurry.

The one or more H₃O⁺ ion donor used for the preparation of surface reacted calcium carbonate may be any strong acid, medium-strong acid, or weak acid, or mixtures thereof, generating H₃O⁺ ions under the preparation conditions. According to the present invention, the at least one H₃O⁺ ion donor can also be an acid salt, generating H₃O⁺ ions under the preparation conditions.

According to one embodiment, the at least one H₃O⁺ ion donor is a strong acid having a pKₐ of 0 or less at 20°C.

According to another embodiment, the at least one H₃O⁺ ion donor is a medium-strong acid having a pKₐ value from 0 to 2.5 at 20°C. If the pKₐ at 20°C is 0 or less, the acid is preferably selected from sulphuric acid, hydrochloric acid, or mixtures thereof. If the pKₐ at 20°C is from 0 to 2.5, the H₃O⁺ ion donor is preferably selected from H₂SO₃, H₃PO₄, oxalic acid, or mixtures thereof. The at least one H₃O⁺ ion donor can also be an acid salt, for example, HSO₄⁻ or H₂PO₄⁻, being at least partially neutralized by a corresponding cation such as Li⁺, Na⁺ or K⁺, or HPO₄²⁻, being at least partially neutralised by a corresponding cation such as Li⁺, Na⁺, K⁺, Mg²⁺ or Ca²⁺. The at least one H₃O⁺ ion donor can also be a mixture of one or more acids and one or more acid salts.

According to still another embodiment, the at least one H₃O⁺ ion donor is a weak acid having a pKₐ value of greater than 2.5 and less than or equal to 7, when measured at 20°C, associated with the ionisation of the first available hydrogen, and having a corresponding anion, which is capable of forming water-soluble calcium salts. Subsequently, at least one water-soluble salt, which in the case of a hydrogen-containing salt has a pKₐ of greater than 7, when measured at 20°C, associated with the ionisation of the first available hydrogen, and the salt anion of which is capable of forming water-insoluble calcium salts, is additionally provided. According to the preferred embodiment, the weak acid has a pKₐ value from greater than 2.5 to 5 at 20°C, and more preferably the weak acid is selected from the group consisting of acetic acid, formic acid, propanoic acid, and mixtures thereof. Exemplary cations of said water-soluble salt are selected from the group consisting of potassium, sodium, lithium and mixtures thereof. In a more preferred embodiment, said cation is sodium or potassium. Exemplary anions of said water-soluble salt are selected from the group consisting of phosphate, dihydrogen phosphate, monohydrogen phosphate, oxalate, silicate, mixtures thereof and hydrates thereof. In a more preferred embodiment, said anion is selected from the group consisting of phosphate, dihydrogen phosphate, monohydrogen phosphate, mixtures thereof and hydrates thereof. In a most preferred embodiment, said anion is selected from the group consisting of dihydrogen phosphate, monohydrogen phosphate, mixtures thereof and hydrates thereof. Water-soluble salt addition may be performed dropwise or in one step. In the case of drop wise addition, this addition preferably takes place within a time period of 10 minutes. It is more preferred to add said salt in one step.

According to one embodiment of the present invention, the at least one H₃O⁺ ion donor is selected from the group consisting of hydrochloric acid, sulphuric acid, sulphurous acid, phosphoric acid, citric acid, oxalic acid, acetic acid, formic acid, and mixtures thereof. Preferably the at least one H₃O⁺ ion donor is selected from the group consisting of hydrochloric acid, sulphuric acid, sulphurous acid, phosphoric acid, oxalic acid, H₂PO₄⁻, being at least partially neutralised by a corresponding cation such as Li⁺, Na⁺ or K⁺, HPO₄²⁻, being at least partially neutralised by a corresponding cation such as Li⁺, Na⁺, K⁺, Mg²⁺, or Ca²⁺ and mixtures thereof, more preferably the at least one acid is selected from the group consisting of hydrochloric acid, sulphuric acid, sulphurous acid, phosphoric acid, oxalic acid, or mixtures thereof, and most preferably, the at least one H₃O⁺ ion donor is phosphoric acid.

The one or more H₃O⁺ ion donor can be added to the suspension as a concentrated solution or a more diluted solution. Preferably, the molar ratio of the H₃O⁺ ion donor to the natural or precipitated calcium carbonate is from 0.01 to 4, more preferably from 0.02 to 2, even more preferably 0.05 to 1 and most preferably 0.1 to 0.58.

As an alternative, it is also possible to add the H₃O⁺ ion donor to the water before the natural or precipitated calcium carbonate is suspended.

In a next step, the natural or precipitated calcium carbonate is treated with carbon dioxide. If a strong acid such as sulphuric acid or hydrochloric acid is used for the H₃O⁺ ion donor treatment of the natural or precipitated calcium carbonate, the carbon dioxide is automatically formed. Alternatively or additionally, the carbon dioxide can be supplied from an external source.

H₃O⁺ ion donor treatment and treatment with carbon dioxide can be carried out simultaneously which is the case when a strong or medium-strong acid is used. It is also possible to carry out H₃O⁺ ion donor treatment first, e.g. with a medium strong acid having a pKₐ in the range of 0 to 2.5 at 20°C, wherein carbon dioxide is formed in situ, and thus, the carbon dioxide treatment will automatically be carried out simultaneously with the H₃O⁺ ion donor treatment, followed by the additional treatment with carbon dioxide supplied from an external source.

Preferably, the concentration of gaseous carbon dioxide in the suspension is, in terms of volume, such that the ratio (volume of suspension):(volume of gaseous CO₂) is from 1:0.05 to 1:20, even more preferably 1:0.05 to 1:5.

In a preferred embodiment, the H₃O⁺ ion donor treatment step and/or the carbon dioxide treatment step are repeated at least once, more preferably several times. According to one embodiment, the at least one H₃O⁺ ion donor is added over a time period of at least about 5 min, preferably at least about 10 min, typically from about 10 to about 20 min, more preferably about 30 min, even more preferably about 45 min, and sometimes about 1 h or more.

Subsequent to the H₃O⁺ ion donor treatment and carbon dioxide treatment, the pH of the aqueous suspension, measured at 20°C, naturally reaches a value of greater than 6.0, preferably greater than 6.5, more preferably greater than 7.0, even more preferably greater than 7.5, thereby preparing the surface-reacted natural or precipitated calcium carbonate as an aqueous suspension having a pH of greater than 6.0, preferably greater than 6.5, more preferably greater than 7.0, even more preferably greater than 7.5.

Further details about the preparation of the surface-reacted natural calcium carbonate are disclosed in WO 00/39222 A1, WO 2004/083316 A1, WO 2005/121257 A2, WO 2009/074492 A1, EP 2 264 108 A1, EP 2 264 109 A1 and US 2004/0020410 A1, the content of these references herewith being included in the present application.

Similarly, surface-reacted precipitated calcium carbonate is obtained. As can be taken in detail from WO 2009/074492 A1, surface-reacted precipitated calcium carbonate is obtained by contacting precipitated calcium carbonate with H₃O⁺ ions and with anions being solubilized in an aqueous medium and being capable of forming water-insoluble calcium salts, in an aqueous medium to form a slurry of surface-reacted precipitated calcium carbonate, wherein said surface-reacted precipitated calcium carbonate comprises an insoluble, at least partially crystalline calcium salt of said anion formed on the surface of at least part of the precipitated calcium carbonate.

Said solubilized calcium ions correspond to an excess of solubilized calcium ions relative to the solubilized calcium ions naturally generated on dissolution of precipitated calcium carbonate by H₃O⁺ ions, where said H₃O⁺ ions are provided solely in the form of a counterion to the anion, i.e. via the addition of the anion in the form of an acid or non-calcium acid salt, and in absence of any further calcium ion or calcium ion generating source.

Said excess solubilized calcium ions are preferably provided by the addition of a soluble neutral or acid calcium salt, or by the addition of an acid or a neutral or acid non-calcium salt which generates a soluble neutral or acid calcium salt in situ.

Said H₃O⁺ ions may be provided by the addition of an acid or an acid salt of said anion, or the addition of an acid or an acid salt which simultaneously serves to provide all or part of said excess solubilized calcium ions.

In a further preferred embodiment of the preparation of the surface-reacted natural or precipitated calcium carbonate, the natural or precipitated calcium carbonate is reacted with the acid and/or the carbon dioxide in the presence of at least one compound selected from the group consisting of silicate, silica, aluminium hydroxide, earth alkali aluminate such as sodium or potassium aluminate, magnesium oxide, or mixtures thereof. Preferably, the at least one silicate is selected from an aluminium silicate, a calcium silicate, or an earth alkali metal silicate. These components can be added to an aqueous suspension comprising the natural or precipitated calcium carbonate before adding the acid and/or carbon dioxide.

Alternatively, the silicate and/or silica and/or aluminium hydroxide and/or earth alkali aluminate and/or magnesium oxide component(s) can be added to the aqueous suspension of natural or precipitated calcium carbonate while the reaction of natural or precipitated calcium carbonate with an acid and carbon dioxide has already started. Further details about the preparation of the surface-reacted natural or precipitated calcium carbonate in the presence of at least one silicate and/or silica and/or aluminium hydroxide and/or earth alkali aluminate component(s) are disclosed in WO 2004/083316 A1, the content of this reference herewith being included in the present application.

The surface-reacted calcium carbonate can be kept in suspension, optionally further stabilised by a dispersant. Conventional dispersants known to the skilled person can be used. A preferred dispersant is comprised of polyacrylic acids and/or carboxymethylcelluloses.

Alternatively, the aqueous suspension described above can be dried, thereby obtaining the solid (i.e. dry or containing as little water that it is not in a fluid form) surface-reacted natural or precipitated calcium carbonate in the form of granules or a powder.

The surface-reacted calcium carbonate may have different particle shapes, such as e.g. the shape of roses, golf balls and/or brains.

In a preferred embodiment, the surface-reacted calcium carbonate has a specific surface area of from 15 m²/g to 200 m²/g, preferably from 27 m²/g to 180 m²/g, more preferably from 30 m²/g to 160 m²/g, even more preferably from 45 m²/g to 150 m²/g, most preferably from 48 m²/g to 140 m²/g, measured using nitrogen and the BET method. For example, the surface-reacted calcium carbonate has a specific surface area of from 75 m²/g to 100 m²/g, measured using nitrogen and the BET method. The BET specific surface area in the meaning of the present invention is defined as the surface area of the particles divided by the mass of the particles. As used therein the specific surface area is measured by adsorption using the BET isotherm (ISO 9277:1995) and is specified in m²/g.

It is furthermore preferred that the surface-reacted calcium carbonate particles have a volume median grain diameter *d*₅₀(vol) of from 1 to 75 µm, preferably from 2 to 50 µm, more preferably 3 to 40 µm, even more preferably from 4 to 30 µm, and most preferably from 4 to 15 µm.

It may furthermore be preferred that the surface-reacted calcium carbonate particles have a grain diameter *d*₉₈(vol) of from 2 to 150 µm, preferably from 4 to 100 µm, more preferably 6 to 80 µm, even more preferably from 8 to 60 µm, and most preferably from 10 to 30 µm.

The value *dₓ* represents the diameter relative to which x % of the particles have diameters less than *dₓ*. This means that the *d*₉₈ value is the particle size at which 98 % of all particles are smaller. The *d*₉₈ value is also designated as "top cut". The d*ₓ* values may be given in volume or weight percent. The *d*₅₀(wt) value is thus the weight median particle size, i.e. 50 wt% of all grains are smaller than this particle size, and the *d*₅₀ (vol) value is the volume median particle size, i.e. 50 vol.% of all grains are smaller than this particle size.

Volume median grain diameter *d*₅₀ was evaluated using a Malvern Mastersizer 2000 Laser Diffraction System. The *d*₅₀ or *d*₉₈ value, measured using a Malvern Mastersizer 2000 Laser Diffraction System, indicates a diameter value such that 50 % or 98 % by volume, respectively, of the particles have a diameter of less than this value. The raw data obtained by the measurement are analysed using the Mie theory, with a particle refractive index of 1.57 and an absorption index of 0.005.

The weight median grain diameter is determined by the sedimentation method, which is an analysis of sedimentation behaviour in a gravimetric field. The measurement is made with a Sedigraph^{™} 5100 or 5120, Micromeritics Instrument Corporation. The method and the instrument are known to the skilled person and are commonly used to determine grain size of fillers and pigments. The measurement is carried out in an aqueous solution of 0.1 wt% Na₄P₂O₇. The samples were dispersed using a high speed stirrer and sonicated.

The processes and instruments are known to the skilled person and are commonly used to determine grain size of fillers and pigments.

The specific pore volume is measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 µm (~ nm). The equilibration time used at each pressure step is 20 seconds. The sample material is sealed in a 5 cm³ chamber powder penetrometer for analysis. The data are corrected for mercury compression, penetrometer expansion and sample material compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 35(5), 1996, p. 1753-1764).

The total pore volume seen in the cumulative intrusion data can be separated into two regions with the intrusion data from 214 µm down to about 1 - 4 µm showing the coarse packing of the sample between any agglomerate structures contributing strongly. Below these diameters lies the fine interparticle packing of the particles themselves. If they also have intraparticle pores, then this region appears bi-modal, and by taking the specific pore volume intruded by mercury into pores finer than the modal turning point, i.e. finer than the bi-modal point of inflection, the specific intraparticle pore volume is defined. The sum of these three regions gives the total overall pore volume of the powder, but depends strongly on the original sample compaction/settling of the powder at the coarse pore end of the distribution.

By taking the first derivative of the cumulative intrusion curve the pore size distributions based on equivalent Laplace diameter, inevitably including pore-shielding, are revealed. The differential curves clearly show the coarse agglomerate pore structure region, the interparticle pore region and the intraparticle pore region, if present. Knowing the intraparticle pore diameter range it is possible to subtract the remainder interparticle and interagglomerate pore volume from the total pore volume to deliver the desired pore volume of the internal pores alone in terms of the pore volume per unit mass (specific pore volume). The same principle of subtraction, of course, applies for isolating any of the other pore size regions of interest.

Preferably, the surface-reacted calcium carbonate has an intra-particle intruded specific pore volume in the range from 0.1 to 2.3 cm³/g, more preferably from 0.2 to 2.0 cm³/g, especially preferably from 0.4 to 1.8 cm³/g and most preferably from 0.6 to 1.6 cm³/g, calculated from mercury porosimetry measurement.

The intra-particle pore size of the surface-reacted calcium carbonate preferably is in a range of from 0.004 to 1.6 µm, more preferably in a range of between 0.005 to 1.3 µm, especially preferably from 0.006 to 1.15 µm and most preferably of 0.007 to 1.0 µm, e.g. 0.004 to 0.510 µm determined by mercury porosimetry measurement.

### The aquatic pesticide

In addition to the surface-reacted calcium carbonate, the composition of the present invention comprises an aquatic pesticide comprising rotenone.

Rotenone ((2*R*,6a*S*,12a*S*)-1,2,6,6a,12,12a-hexahydro-2-isopropenyl-8,9-dimethoxychromeno[3,4-*b*]furo(2,3-h)chromen-6-one) is an odourless, colourless, crystalline ketonic chemical compound used as a broad-spectrum insecticide, piscicide, and pesticide. It occurs naturally in the seeds and stems of several plants, such as the jicama vine plant, and the roots of several members of *Fabaceae* (cf. Wikipedia contributors, "Rotenone", Wikipedia, The Free Encyclopedia, September 24, 2015). Rotenone is insoluble in water and has the following structural formula:

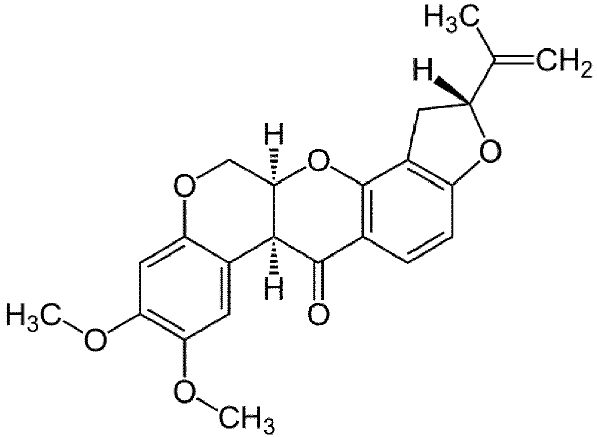

Rotenone works by interfering with the electron transport chain in mitochondria. More precisely, it inhibits the transfer of electrons from iron-sulphur centres in complex I to ubiquinone. This interferes with NADH during the creation of usable cellular energy (ATP). Complex I is unable to pass off its electron to CoQ, creating a back-up of electrons within the mitochondrial matrix. Cellular oxygen is reduced to the radical, creating a reactive oxygen species, which can damage DNA and other components of the mitochondria. The compound decomposes to rotenolone when exposed to sunlight and usually has an activity of six days in the environment (cf. Wikipedia contributors, "Rotenone", Wikipedia, The Free Encyclopedia, September 24, 2015).

Rotenone may be produced by extraction from the roots and stems of the tropical and subtropical plant species *Lonchocarpus* and *Derris.* The root extract from the cube plant or lancepod (*Lonchocarpus utilis*), and barbasco (*Lonchocarpus urucu*) is designated as cube resin. The root extract from the tuba plant (*Derris elliptica*), and jewel vine (*Derris involuta*) is designated as derris resin or derris root.

The aquatic pesticide may comprise rotenone, a rotenone-containing plant extract, a formulation comprising rotenone as the active ingredient, or combinations thereof. According to one embodiment the aquatic pesticide consists of rotenone. According to another embodiment, the aquatic pesticide comprises a rotenone-containing plant extract, preferably selected from cube resin and/or derris resin. According to still another embodiment, the aquatic pesticide comprises a formulation comprising rotenone as the active ingredient. The aquatic pesticide can also be a combination of rotenone with a rotenone-containing plant extract and/or a formulation comprising rotenone as the active ingredient.

Suitable pesticide formulations comprising rotenone as the active ingredient are known to the skilled person. Such formulations often contain organic solvents and further additives in order to facilitate the application of the hydrophobic compound rotenone to aquatic systems. Examples are CFT Legumine^{®}, TIFA Chem Fish^{®}, or Prentox Prenfish^{®}.

CFT Legumine^{®} is a commercial product formulation containing rotenone as the active ingredient. The new formulation "CFT Legumine^{®} 3.3 %", commercially available from Veso Pharmacy, Norway, contains the following ingredients:
8.05 wt.-% (3.3 wt.-% active ingredient) cube resin, containing 41% rotenone (extracted raw material),
59.95 wt.-% diethylene-glycol-monoethyl ether (solvent),
10.0 wt.-% cyclic trimethylolpropane (solvent),
20.0 wt.-% Fennedefo 99 (tall oil ester, emulsifier), and
2.0 wt.-% calcium alkyl benzene sulphonate (emulsifier),
wherein the wt.-% are based on the total weight of CFT Legumine 3.3%.

TIFA Chem Fish^{®} are commercial product formulations containing rotenone and other cube resins as the active ingredient and are commercially available from TIFA International LLC, USA. For example, TIFA Chem Fish^{®} Regular may comprise 5 wt.-% rotenone and 5 wt.-% other cube extracts as the active ingredients, based on the total weight of the formulation, and TIFA Chem Fish^{®} Synergized may comprise 2.5 wt.-% rotenone, 2.5 wt.-% other cube extracts, and 2.5 wt.-% piperonyl butoxide as the active ingredients, based on the total weight of the formulation.

Prentox Prenfish^{®} is a commercial product formulation containing rotenone and other cube resins as the active ingredient. Said formulation is commercially available from Prentiss Inc., USA, and may comprise the following ingredients: 5 wt.-% rotenone, 10 wt.-% other cube resins, 9.9 wt.-% naphthalene, 1.7 wt.-% 1,2,4-trimethylbenzene, acetone, and emulsifiers, wherein the wt.-% are based on the total weight of the formulation.

According to one embodiment the aquatic pesticide is selected from the group consisting of rotenone, a rotenone-containing plant extract, a formulation comprising rotenone, and mixtures thereof. According to a preferred embodiment the aquatic pesticide is selected from the group consisting of rotenone, cube resin, derris resin, CFT Legumine^{®}, TIFA Chem Fish^{®}, Prentox Prenfish^{®}, Cuberol^{®}, Fish Tox^{®}, Noxfire^{®}, Rotacide^{®}, Sinid^{®}, Tox-R^{®}, Curex Flea Duster^{®}, Derrin, Cenol Garden Dust^{®}, Chem-Mite^{®}, Cibe Extract, Green Cross Warble Powder^{®}, and mixtures thereof.

According to one embodiment of the present invention, the aquatic pesticide is CFT Legumine^{®}, and more preferably CFT Legumine^{®} 3.3%.

According to one embodiment the aquatic pesticide has an absolute water solubility at 20°C of less than 10 g/l, preferably less than 1 g/l, more preferably less than 0.1 g/l, and most preferably less than 0.01 The "absolute water solubility" of a compound is to be understood as the maximum concentration of a compound in water where one can observe a single phase mixture at 20°C under equilibrium conditions. The absolute water solubility is given in g compound per 100 g water.

### The inventive composition

According to one aspect of the present invention a composition comprising an aquatic pesticide and a surface-reacted calcium carbonate is provided, wherein the aquatic pesticide comprises rotenone, and the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source.

According to one embodiment, the composition of the present invention comprises the aquatic pesticide in an amount from 0.001 to 99.999 wt.-%, preferably from 0.01 to 90 wt.-%, more preferably from 0.05 to 80 wt.-%, and most preferably from 0.1 to 70 wt.-%, based on the total weight of the composition.

According to one embodiment, the composition of the present invention comprises the surface-reacted calcium carbonate in an amount from 0.01 to 99.999 wt.-%, preferably from 0.1 to 90 wt.-%, more preferably from 0.5 to 80 wt.-%, and most preferably from 1 to 70 wt.-%, based on the total weight of the composition.

According to one embodiment the aquatic pesticide and the surface-reacted calcium carbonate are present in a weight ratio from 0.05:1 to 20:1, preferably from 1:1 to 15:1, more preferably from 1.5:1 to 10:1, and most preferably from 2:1 to 1:2.

According to one embodiment, the composition of the present invention comprises the rotenone in an amount from 0.001 to 99.999 wt.-%, preferably from 0.01 to 90 wt.-%, more preferably from 0.05 to 80 wt.-%, and most preferably from 0.1 to 70 wt.-%, based on the total weight of the composition.

According to one embodiment the rotenone and the surface-reacted calcium carbonate are present in a weight ratio from 0.05:1 to 20:1, preferably from 1:1 to 15:1, more preferably from 1.5:1 to 10:1, and most preferably from 2:1 to 1:2.

According to an exemplary embodiment, the composition comprises from 5 to 15 wt.-%, preferably 10 wt.-%, based on the total weight of the composition, of the aquatic pesticide, and from 1 to 10 wt.-%, preferably 4 wt.-%, based on the total weight of the composition, of the surface-reacted calcium carbonate.

According to one embodiment of the present invention, the composition is in form of a suspension, preferably in form of an aqueous suspension. The term "aqueous" suspension refers to a system, wherein the liquid phase or solvent of the suspension comprises, preferably consists of, water. However, said term does not exclude that the aqueous suspension comprises an organic solvent selected from the group comprising alcohols such as methanol, ethanol, isopropanol, carbonyl-group containing solvents such as ketones, e.g. acetone or aldehydes, esters such as isopropyl acetate, carboxylic acids such as formic acid, sulphoxides such as dimethyl sulphoxide, and mixtures thereof. If the aqueous suspension comprises an organic solvent, the aqueous suspension comprises the organic solvent in an amount up to 40.0 wt.-%, preferably from 1.0 to 30.0 wt.-%, and most preferably from 1.0 to 25.0 wt.-%, based on the total weight of the liquid phase of the aqueous suspension. For example, the liquid phase of the aqueous suspension consists of water. If the liquid phase of the aqueous suspension consists of water, the water to be used can be any water available such as tap water and/or deionised water.

According to one embodiment of the present invention, the composition is in form of an aqueous suspension, preferably having a solid content from 0.1 to 85 wt.-%, more preferably from 1 to 50 wt.-%, and most preferably from 5 to 25 wt.-%, based on the total weight of the aqueous suspension. The solids content of the aqueous suspensions can be adjusted by the methods known to the skilled person. To adjust the solids content of an aqueous suspension, the aqueous suspension may be partially dewatered by a settling, filtration, centrifugation or thermal separation process.

According to one embodiment of the present invention, the composition has a Brookfield viscosity from 0.1 to 10 000 mPa·s at 25°C, preferably from 1 to 1 000 mPa·s at 25°C, more preferably from 5 to 800 mPa·s at 25°C, and most preferably from 10 to 600 mPa·s at 25°C.

According to one embodiment of the present invention, the composition has a density from 1.0 to 2.7 g/cm³, preferably from 1.2 to 2.5 g/cm³, more preferably from 1.4 to 2.2 g/cm³, and most preferably from 1.6 to 2.0 g/cm³.

According to another aspect of the present invention, a method for preparing a composition according to the present invention is provided, comprising the following steps:
(I) providing a solution of an aquatic pesticide, wherein the aquatic pesticide comprises rotenone,
(II) providing a surface-reacted calcium carbonate in form of a dry solid, wherein the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source, and
(III) mixing the solution of step (I) and the particulate material of step (II).

The mixing of method step (III) can be carried out by any method known in the art, and the skilled person will adapt the mixing conditions such as the mixing speed and temperature according to his process equipment. For example, the mixing may take place by means of a ploughshare mixer. Ploughshare mixers function by the principle of a fluidized bed produced mechanically. Ploughshare blades rotate close to the inside wall of a horizontal cylindrical drum and convey the components of the mixture out of the product bed and into the open mixing space. The fluidized bed produced mechanically ensures intense mixing of even large batches in a very short time. Choppers and/or dispersers are used to disperse lumps in a dry operation. Equipment that may be used in the inventive process is available, for example, from Gebrüder Lödige Maschinenbau GmbH, Germany, or from Silverson, U.S.A. Furthermore, a tubular mixing apparatus, for example, from Ystral GmbH, Ballrechten-Dottingen, Germany may be used. Other equipment that may be used in the inventive process is a MEGATRON® Inline homogenizer from Kinematika AG, Switzerland.

The mixing step can also be carried out by shaking, stirring, agitating, ultrasonication, or inducing a turbulent or laminar flow by means such as baffles or lamellas. According to one embodiment, the mixing step is carried out manually by stirring the solution of step (I) and the surface-reacted calcium carbonate of step (II), for example, by using a stick or stirrer. According to another embodiment, the mixing step is carried out by shaking the solution of step (I) and the surface-reacted calcium carbonate of step (II) in a container, for example, a flask.

It is also within the confines of the present invention that additional water may be introduced during step III), for example, in order to control and/or maintain and/or achieve the desired solids content or Brookfield viscosity of the obtained composition.

According to one embodiment, step (III) comprises the steps of
a1) mixing the solution of step (I) with the surface-reacted calcium carbonate of step (II), and
a2) mixing the suspension obtained in step a1) with water.

According to another embodiment, step (III) comprises the steps of
b1) mixing the solution of step (I) with water, and
b2) mixing the suspension obtained in step b1) with the surface-reacted calcium carbonate of step (II).

The inventors of the present invention surprisingly found that the composition of the present invention has an increased density. This, once mixed with water, may improve the sedimentation of the composition, may allow the composition to sink to specific spots at the application area and may increase the treatment efficiency. In particular, it was found that the composition of the present invention can sink into locations of aquatic systems, which are difficult to access, such as small brooks and seeps of rivers, where significant outlets of ground water are found. Furthermore, the risk that the pesticide is taken downstream of a river is significantly reduced. Thus, the composition of the present invention may allow, for example, a more effective treatment of difficult spots within aquatic systems. For example, it would be possible to treat lakes more easily and efficiently because the composition sinks down to the bottom of lake, while prior art formulation often require that they are applied at a certain depth level in order to ensure that they reach the ground of the lake. Moreover, it was found that the composition of the present invention can form a stable suspension, prior to mixing with water, which does not settle quickly, and thus, can improve the handling of the composition. For example, it can be sprayed out on water through a pump and a hose without the need of stirring it continuously.

Furthermore, the surface-reacted calcium carbonate is non-toxic and does not affect the efficiency of the aquatic pesticide in a negative way. The surface-reacted calcium carbonate can also be capable of associating and transporting the aquatic pesticide. The association preferably is an adsorption onto the surface of the surface-reacted calcium carbonate, be it the outer or the inner surface of the particles or an absorption into the particles, which is possible due to their porosity. This may allow a slow release of the aquatic pesticide, and hence, may further improve the treatment with the aquatic pesticide. Furthermore, the inventors found that the aquatic pesticide degrades on a slower timescale. Without being bound to any theory, the inventors believe that in the inventive composition the aquatic pesticide is at least partially protected from light and oxygen.

Due to the intra and interpore structure of the surface-reacted calcium carbonate, it can be a superior agent to deliver previously ad/absorbed materials over time relative to common materials having similar specific surface areas. Thus, generally, any agent fitting into the intra and/or inter particle pores of the surface-reacted calcium carbonate is suitable to be transported by the particulate material. For example, materials such as those selected from the group comprising pharmaceutically active agents, biologically active agents, disinfecting agents, preservatives, or lures can be used. According to one embodiment, at least one active agent is associated with the surface-reacted calcium carbonate. According to a preferred embodiment the active agent is at least one additional aquatic pesticide, preferably selected from the group consisting of antimycin A, copper sulphate, chlorine, chloramines, aluminium, sulphuric acid, and mixtures thereof.

In some embodiments, for example, when the control of multiple pests is desired, the composition of the present invention may contain a combination of an aquatic pesticide comprising rotenone with one or more natural or synthetic aquatic pesticides.

According to one embodiment the composition of the present invention further comprises an additional pesticide selected from the group consisting of antimycin A, copper sulphate, chlorine, chloramines, aluminium, sulphuric acid, carbaryl, lindane, thiram, piperonyl butoxide, pyrethrins, quassia, and mixtures thereof. The composition of the present invention may comprise the additional pesticide in an amount from 0.001 to 25 wt.-%, preferably from 0.01 to 20 wt.-%, more preferably from 0.05 to 15 wt.-%, and most preferably from 0.1 to 10 wt.-%, based on the total weight of the composition.

### Use and method for controlling an aquatic pest

According to still another aspect of the present invention use of a composition for controlling an aquatic pest is provided, wherein the composition comprises an aquatic pesticide and a surface-reacted calcium carbonate, and wherein the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source. It is to be understood that the embodiments relating to the inventive composition described above and in the following also apply to the inventive use.

The aquatic pest to be controlled can be a water plant, a crustacean, an invasive species, an insect, a nematode, a mollusc, a fish, a parasite and/or a pathogen. According to one embodiment the aquatic pest is a fish and/or a parasite. Within the context of the present invention, the term "pathogen" designates a pathogen in general, and more preferably a fish pathogen, i.e. an infectious organism capable of proliferation that causes a disease or illness in a fish. Examples of pathogens are fungi, oomycetes, bacteria, viruses, viroids, virus-like organisms, phytoplasma or protozoa.

According to one embodiment, the aquatic pest to be controlled is a pest fish and/or a fish parasite.

For the purpose of the present invention, the term "pest fish" refers to a fish species that is not native to an area and has potential negative social, economic or environmental impacts. Examples of a pest fish in Western Europe are the invasive alien fish species *Pseudorasbora parva* and/or *Cyprinus carpio.*

A "parasite" in the meaning of the present invention is an organism that has sustained contact with another organism, for example, a fish, to the detriment of said host organism. According to one embodiment of the present invention, the parasite is a fish parasite, and preferably a fish parasite selected from the group consisting of *Gyrodactylus salaris, Ichthyophthirius multifiliis*, cryptocaryon, velvet disease, *Brooklynella hostilis*, *Glugea*, *Ceratomyxa shasta*, *Kardoa thyrsites*, *Tetracapsuloides bryosalmonae*, *Cymothoa exigua*, flukes, carp lice, salmon lice, and mixtures thereof, and most preferably *Gyrodactylus salaries*.

*Gyrodactylus salaris* is a small monogenean ectoparasite (about 0.5 mm long) which mainly lives on the skin of freshwater fish, especially Atlantic salmon. This species is a leech-like parasite that has been implicated in some diminution of Atlantic salmon populations in the Norwegian fjords. Other species that can be parasitized include rainbow trout, Arctic char, North American brook trout, grayling, North American lake trout and brown trout. Attachment of the parasite can cause large wounds and feeding can damage the epidermis, allowing secondary infection.
*G. salaris* can build up to very high infection intensity of several thousand parasites on a single salmon parr. (cf. Wikipedia contributors, "Gyrodactylus salaris", Wikipedia, The Free Encyclopedia, October 1, 2015).

According to one embodiment the pest is controlled by controlling the host of the pest, wherein the host is preferably a fish. In other words, the pest is controlled by preventing, treating, reducing, eliminating, or eradicating the host.

According to one embodiment, use of a composition for controlling an aquatic pest is provided, wherein the composition comprises a hydrophobic aquatic pesticide, preferably an aquatic pesticide comprising rotenone, and a surface-reacted calcium carbonate, wherein the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source. Preferably the aquatic pest is a pest fish and/or a fish parasite, more preferably the aquatic pest is selected from the group consisting of *Pseudorasbora parva, Cyprinus carpio, Gyrodactylus salaris, Ichthyophthirius multifiliis*, cryptocaryon, velvet disease, *Brooklynella hostilis*, *Glugea*, *Ceratomyxa shasta*, *Kudoa thyrsites*, *Tetracapsuloides bryosalmonae*, *Cymothoa exigua*, flukes, carp lice, salmon lice, and mixtures thereof, and most preferably the aquatic pest is *Gyrodactylus salaries*.

According to still another aspect of the present invention, a method of controlling an aquatic pest is provided, wherein the method comprises the step of applying a composition comprising an aquatic pesticide and a surface-reacted calcium carbonate onto at least a part of an aquatic system, and wherein the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source.

The aquatic system may be a natural aquatic ecosystem or an artificial aquatic system. According to one embodiment the aquatic system is a catch basin, a pond, a breeding tank, a lake, a bay, a wet land, a marsh, a swamp, a tidal basin, a lagoon, a storm water retention pond, a sound, a creek, a stream, a river, an ocean, a ditch, a swale, a sewage treatment system, a pothole, a tree hole, a rock hole, a water reservoir, a river, a watercourse, or a sewerage system.

The composition of the present invention can be applied by any suitable method and equipment known to the skilled person. Suitable application methods include sprinkling, spraying, pouring, pumping, or injecting.

A skilled person will apply the composition of the present invention according to the need, in accordance with the aquatic system to be treated and in accordance with the pest to be controlled, while preparing efficient concentrations and employing suitable dilutions. Optimal use of the inventive composition may involve repeated application. However, it is preferred that the composition is only applied once.

According to one embodiment, the composition of the present invention is applied in an amount such that the concentration of rotenone in the treated aquatic system is from 0.001 to 10 mg/l, preferably from 0.005 to 8 mg/l, more preferably from 0.01 to 4 mg/l, and most preferably from 0.1 to 1 mg/l.

The inventive method of controlling an aquatic pest can be combined with other methods such as dewatering and/or physical removal of the pest or infected host. Examples for dewatering techniques are damming, water by-passing, or water draining. Examples of physical removal techniques are electro fishing, or removal by nets, traps or seines.

The scope and interest of the invention will be better understood based on the following figures and examples which are intended to illustrate certain embodiments of the present invention and are non-limitative.

### Description of the figures

Fig. 1 is a picture series showing the settling behaviour of an inventive composition (Fig. 1A) and a comparative composition (Fig. 1B).
Fig. 2 shows the settling behaviour of an inventive composition (left) and a comparative composition (right) 60 min after application.
Fig. 3 is a graph showing the concentration of rotenone over time after application of an inventive composition and a comparative composition.

### Examples

### 1. Measurement methods

In the following, measurement methods implemented in the examples are described.

### BET specific surface area (SSA)

The BET specific surface area was measured via the BET process according to ISO 9277 using nitrogen, following conditioning of the sample by heating at 250°C for a period of 30 minutes. Prior to such measurements, the sample was filtered, rinsed and dried at 110°C in an oven for at least 12 hours.

### Particle size distribution

Volume determined median particle size *d*₅₀(vol) and the volume determined top cut particle size *d*₉₈(vol) was evaluated using a Malvern Mastersizer 2000 Laser Diffraction System (Malvern Instruments Plc., Great Britain). The *d*₅₀(vol) or *d*₉₈(vol) value indicates a diameter value such that 50 % or 98 % by volume, respectively, of the particles have a diameter of less than this value. The raw data obtained by the measurement was analysed using the Mie theory, with a particle refractive index of 1.57 and an absorption index of 0.005.

The weight determined median particle size *d*₅₀(wt) was measured by the sedimentation method, which is an analysis of sedimentation behaviour in a gravimetric field. The measurement was made with a Sedigraph^{™} 5100 or 5120 of Micromeritics Instrument Corporation, USA. The method and the instrument are known to the skilled person and are commonly used to determine particle size distributions of fillers and pigments. The measurement was carried out in an aqueous solution of 0.1 wt.-% Na₄P₂O₇. The samples were dispersed using a high speed stirrer and sonicated.

### Intra-particle intruded specific pore volume (in cm³/g)

The specific pore volume was measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 µm (~ nm). The equilibration time used at each pressure step was 20 seconds. The sample material was sealed in a 5 cm³ chamber powder penetrometer for analysis. The data were corrected for mercury compression, penetrometer expansion and sample material compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 35(5), 1996, p1753-1764.).

The total pore volume seen in the cumulative intrusion data can be separated into two regions with the intrusion data from 214 µm down to about 1 - 4 µm showing the coarse packing of the sample between any agglomerate structures contributing strongly. Below these diameters lies the fine inter-particle packing of the particles themselves. If they also have intra-particle pores, then this region appears bi modal, and by taking the specific pore volume intruded by mercury into pores finer than the modal turning point, i.e. finer than the bi-modal point of inflection, the specific intra-particle pore volume is defined. The sum of these three regions gives the total overall pore volume of the powder, but depends strongly on the original sample compaction/settling of the powder at the coarse pore end of the distribution.

By taking the first derivative of the cumulative intrusion curve the pore size distributions based on equivalent Laplace diameter, inevitably including pore-shielding, are revealed. The differential curves clearly show the coarse agglomerate pore structure region, the inter-particle pore region and the intra-particle pore region, if present. Knowing the intra-particle pore diameter range it is possible to subtract the remainder inter-particle and inter-agglomerate pore volume from the total pore volume to deliver the desired pore volume of the internal pores alone in terms of the pore volume per unit mass (specific pore volume). The same principle of subtraction, of course, applies for isolating any of the other pore size regions of interest.

### LC-MS

The sample was filtered through a syringe filter (0.2 µm RC (regenerated cellulose)), first 2 ml in waste (condition of the filter), then in vial. Filtered sample were diluted 1:1 with methanol (500 µl sample + 500 µl methanol) and analyzed by LC-MS. The UPLC-MS system used was Acquity UPLC-System®: Binary Solvent Manager, Sample Manager, Column Manager (Waters) and Xevo TQ™ MS System (Waters).

The following UPLC parameters were used:

| Column | AQUITY UPLC® BEH C18 1.7 µm, 2.1 mm x 100 mm |
|---|---|
| Eluent (isocratic) | 70% methanol with 10 mM ammonium acetate (pH 5.0) |
| | 30% water with 10 mM ammonium acetate (pH 5.0) |
| Flow (mL/min) | 0.4 |
| Injection amount (µL) | 5 |
| Temperature (°C) | 45 |
| Analysis time (minutes) | 5 |

The following MS/MS parameters were used:

| | |
|---|---|
| Capillary (kV): | 0.7 |
| Source temperature (C°): | 150 |
| Desolvation temperature (C°): | 500 |
| Desolvation gas flow (L/Hr) N₂: | 1000 |
| Collision gas flow (L/Hr) Ar: | 0.15 |

### 2. Materials

### Surface-reacted calcium carbonate (SRCC 1)

SRCC 1 had a *d*₅₀(vol) = 4.93 µm, *d*₉₈(vol) = 15.0 µm, SSA = 37.8 m²/g with an intra-particle intruded specific pore volume of 0.530 cm³/g (for the pore diameter range of 0.004 to 0. 510 µm).

The feed used for preparing the surface-reacted calcium carbonate was an aqueous suspension of natural ground calcium carbonate from Omya Hustadmarmor, Norway, having a weight determined median particle size *d*₅₀(wt) of 8 µm and a solids content of 40 wt.-%, based on the total weight of the aqueous suspension.

The feed was ground in a Dyno-Mill Multi-Lab mill (W. Bachofen AG) using Verac grinding media with a diameter of 0.7-1.4 mm in form of an aqueous suspension in order to obtain a finer calcium carbonate. The obtained aqueous suspension had a weight determined median particle size *d*₅₀(wt) of 1 µm and a solids content of 18 wt.-%, based on the total weight of the aqueous suspension.

The obtained ground feed suspension was placed in a mixing vessel and while rapidly mixing the suspension, phosphoric acid was added to that suspension in an amount of 9 to 12 wt.-% of active phosphoric acid, based on the dry weight of the ground calcium carbonate. After the addition of the acid, the slurry was stirred for additional 5 minutes, before removing it from the vessel, dewatering it mechanically and drying the resulting filter cake. During acid treatment, carbon dioxide was formed in situ in the aqueous suspension.

The resulting surface-reacted calcium carbonate-comprising mineral material SRCC 1 was in the form of a dry powder.

### CFT Legumine

CFT Legumine 3.3%, commercially available from Veso, Norway.

### 3. Examples

### Example 1

A pre-formulation containing 25.5 wt.-%, based on the total weight of the pre-formulation, of the particulate material SRCC1 in CFT Legumine (Veso) was prepared.

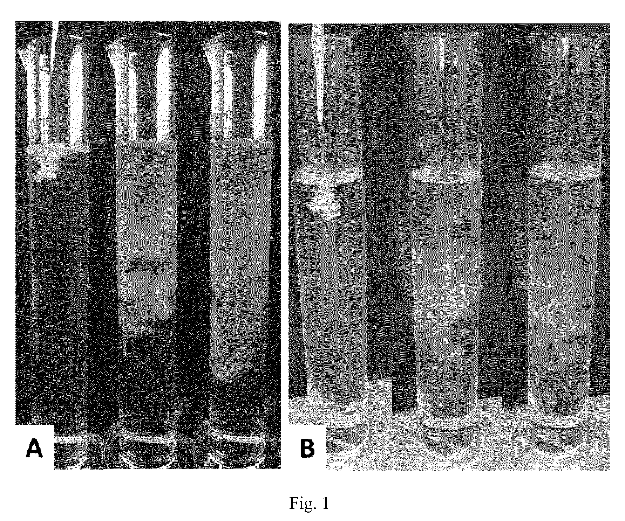

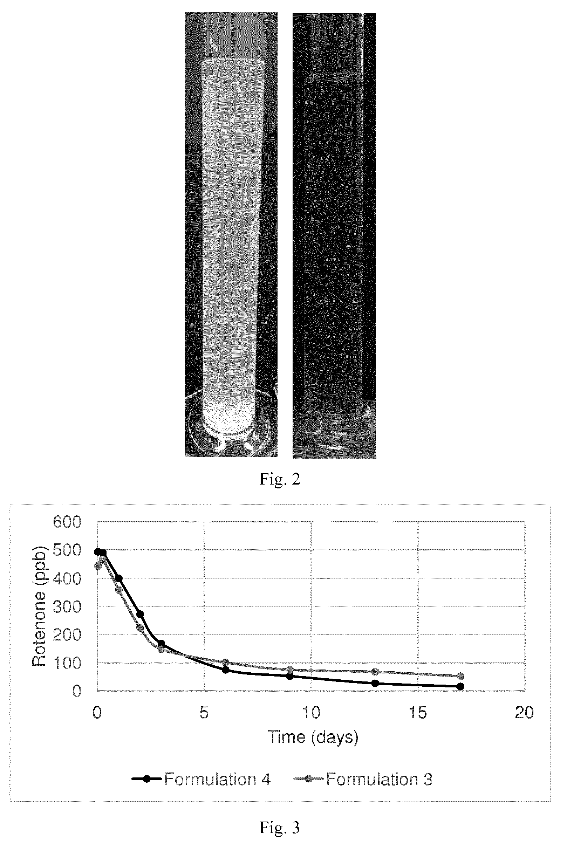

This pre-formulation was mixed 12.5% (v/v) with water, i.e. in a volume ratio of 1:8 (Formulation 1). Subsequently, 1 ml of Formulation 1 was dropped into 1 000 ml of water. As a comparative test, 0.5 ml of a formulation containing CFT Legumine and water in a volume ratio of 1:8 (Formulation 2) was dropped into 500 ml of water. The behaviour of both formulations was inspected visually and is depicted in Figs. 1 and 2.

Figure 1 shows a picture series of the settling behaviour of the inventive and comparative formulation, when dropped in one litre or 500 ml, respectively, of water over time. Fig. 1A shows pictures of Formulation 1 at 2, 30 and 49 seconds after the formulation has been dropped into the water, and Fig. 1B shows pictures of Formulation 2 at 2, 30 and 49 seconds after the formulation has been dropped into the water. As can be gathered from Fig. 2, after 60 min the inventive Formulation 1 has been settled (left picture), while the comparative Formulation 2 has not been settled (right picture).

These results confirm that by combining the surface-reacted calcium carbonate and CFT Legumine a formulation is obtained, which settles in an aqueous environment after a short period of time. Thus, the inventive composition can sink or settle in river beds and gravel in small brooks and seeps, where small fish may hide and would typically escape the treatment with conventional rotenone-comprising formulations.

### Example 2

The concentration of rotenone through time in river simulations was studied by LC/MS.

A pre-formulation containing 25.5 wt.-%, based on the total weight of the pre-formulation, of the particulate material SRCC1 in CFT Legumine (Veso) was prepared.

This pre-formulation was mixed 10% (v/v) with water, i.e. in a volume ratio of 1:10 (Formulation 3). Subsequently, 150 µl of Formulation 3 was dropped into 1 000 ml of water to study the release of rotenone. As a comparative test, 150 µl of a formulation containing CFT Legumine and water in a volume ratio of 1:10 (Formulation 4) was dropped into 1 000 ml of water.

At the times 0, 5, 15, 30, 60, 180, 360, 1 440 and 2 880 minutes, 9 ml were taken from the treated water volume with a syringe and filtered with a 0.2 µm RC membrane filter. The first 5 ml of the filtrate were discarded, and 4 ml were used further for the measurement of rotenone by LC/MS. The results of the LC/MS study are shown in Fig. 3.

As can be gathered from Fig. 3 the concentration of rotenone in water after 5 days is higher in the water volume, which was treated with the inventive Formulation 3. The concentration of rotenone decreases with time for both formulations. Likely this decrease is due to the degradation of rotenone in water. Interestingly, between the measurements on day six and day nine degradation of rotenone for the CFT Legumine alone was faster compared to CFT legumine mixed with SRCC. In fact on day 17 the concentration of rotenone was 3.25 times higher on the beaker with the CFT Legumine mixed with SRCC than in the one with just CFT Legumine.

The LC/MS study showed that the surface-reacted calcium carbonate can provide a reservoir of the CFT Legumine which may reduce the risk of re-infection of the treated river due to escape of small fish into outlet ground waters.

## Claims

1. A composition comprising an aquatic pesticide and a surface-reacted calcium carbonate, wherein
the aquatic pesticide comprises rotenone, and
the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source.

2. The composition of claim 1, wherein the aquatic pesticide has an absolute water solubility at 20°C of less than 10 g/l, preferably less than 1 g/l, more preferably less than 0.1 g/l, and most preferably less than 0.01

3. The composition of any one of the preceding claims, wherein the aquatic pesticide and the surface-reacted calcium carbonate are present in a weight ratio from 0.1:1 to 20:1, preferably from 1:1 to 15:1, more preferably from 1.5:1 to 10:1, and most preferably from 2:1 to 5:1.

4. The composition of any one of the preceding claims, wherein the aquatic pesticide is selected from the group consisting of rotenone, a rotenone-containing plant extract, a formulation comprising rotenone, and mixtures thereof, preferably the aquatic pesticide is selected from the group consisting of rotenone, cube resin, derris resin, CFT Legumine, TIFA Chem Fish, Prentox Prenfish, Cuberol, Fish Tox, Noxfire, Rotacide, Sinid, Tox-R, Curex Flea Duster, Derrin, Cenol Garden Dust, Chem-Mite, Cibe Extract, Green Cross Warble Powder, and mixtures thereof.

5. The composition of any one of the preceding claims, wherein the surface-reacted calcium carbonate has a volume median particle size *d*₅₀ from 1 to 75 µm, preferably from 2 to 50 µm, more preferably from 3 to 40 µm, even more preferably from 4 to 30 µm, and most preferably from 4 to 15 µm.

6. The composition of any one of the preceding claims, wherein the surface-reacted calcium carbonate is obtained by a process comprising the steps of:
(a) providing a suspension of natural ground calcium carbonate or precipitated calcium carbonate,
(b) adding at least one acid having a pKₐ value of 0 or less at 20°C or having a pKₐ value from 0 to 2.5 at 20°C to the suspension of step a), and
(c) treating the suspension of step (a) with carbon dioxide before, during or after step (b).

7. The composition of any one of claims 1 to 5, wherein the surface-reacted calcium carbonate is obtained by a process comprising the steps of:
(A) providing a natural ground calcium carbonate or precipitated calcium carbonate,
(B) providing at least one water-soluble acid,
(C) providing gaseous carbon dioxide,
(D) contacting said natural ground calcium carbonate or precipitated calcium carbonate of step (A) with the at least one acid of step (B) and with the carbon dioxide of step (C),
**characterised in that**:
(i) the at least one acid of step (B) has a pKₐ greater than 2.5 and less than or equal to 7 at 20°C, associated with the ionisation of its first available hydrogen, and a corresponding anion is formed on loss of this first available hydrogen capable of forming a water-soluble calcium salt, and
(ii) following contacting the at least one acid with natural ground calcium carbonate or precipitated calcium carbonate, at least one water-soluble salt, which in the case of a hydrogen-containing salt has a pKₐ greater than 7 at 20°C, associated with the ionisation of the first available hydrogen, and the salt anion of which is capable of forming water-insoluble calcium salts, is additionally provided.

8. The composition of any one of the preceding claims, wherein
the natural ground calcium carbonate is selected from the group consisting of marble, chalk, limestone, and mixtures thereof, or
the precipitated calcium carbonate is selected from the group consisting of precipitated calcium carbonates having an aragonitic, vateritic or calcitic crystal form, and mixtures thereof.

9. The composition of any one of the preceding claims, wherein the composition is in form of an aqueous suspension, preferably having a solid content from 1 to 85 wt.-%, more preferably from 5 to 50 wt.-%, and most preferably from 10 to 25 wt.-%, based on the total weight of the aqueous suspension.

10. A method for preparing a composition according to any one of claims 1 to 9, comprising the following steps:
(I) providing a solution of an aquatic pesticide, wherein the aquatic pesticide comprises rotenone,
(II) providing a surface-reacted calcium carbonate in form of a dry solid, wherein the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source, and
(III) mixing the solution of step (I) and the surface-reacted calcium carbonate of step (II).

11. Use of a composition for controlling an aquatic pest,
wherein the composition comprises an aquatic pesticide and a surface-reacted calcium carbonate, and
wherein the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source.

12. The use of claim 11, wherein the aquatic pest is a water plant, a crustacean, an invasive species, an insect, a nematode , a mollusc, a fish, a parasite and/or a pathogen, preferably a fish and/or parasite, more preferably a fish parasite, even more preferably a fish parasite selected from the group consisting of *Gyrodactylus salaris, Ichthyophthirius multifiliis*, cryptocaryon, velvet disease, *Brooklynella hostilis*, *Glugea*, *Ceratomyxa shasta*, *Kudoa thyrsites, Tetracapsuloides bryosalmonae*, *Cymothoa exigua*, flukes, carp lice, salmon lice, and mixtures thereof, and most preferably *Gyrodactylus salaries*.

13. The use of claim 11 or 12, wherein the pest is controlled by controlling the host of the pest, wherein the host is preferably a fish.

14. A method of controlling an aquatic pest,
wherein the method comprises the step of applying a composition comprising an aquatic pesticide and a surface-reacted calcium carbonate onto at least a part of an aquatic system, and
wherein the surface-reacted calcium carbonate is a reaction product of natural ground calcium carbonate or precipitated calcium carbonate with carbon dioxide and one or more H₃O⁺ ion donors, wherein the carbon dioxide is formed in situ by the H₃O⁺ ion donor treatment and/or is supplied from an external source.

15. The method of claim 14, wherein the aquatic system is a catch basin, a pond, a breeding tank, a lake, a bay, a wet land, a marsh, a swamp, a tidal basin, a lagoon, a storm water retention pond, a sound, a creek, a stream, a river, an ocean, a ditch, a swale, a sewage treatment system, a pothole, a tree hole, a rock hole, a water reservoir, a river, a watercourse, or a sewerage system.
